(19) Europäisches Patentamt
European Patent Office
Office européen des brevets

(11) Publication number: **0 245 902 A2**

(12) # EUROPEAN PATENT APPLICATION

(21) Application number: 87200819.8

(22) Date of filing: 29.04.87

(51) Int. Cl.³: **A 01 N 43/86**
C 07 D 279/08, C 07 D 417/04

(30) Priority: 13.05.86 GB 8611618

(43) Date of publication of application:
19.11.87 Bulletin 87/47

(84) Designated Contracting States:
AT BE CH DE ES FR GB GR IT LI LU NL SE

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Gilkerson, Terence**
**11 The Ness**
**Canterbury Kent CT 3NL(GB)**

(72) Inventor: **Jennens, David Clifford**
**13 Thistle Walk**
**Sittingbourne Kent(GB)**

(72) Inventor: **Coombs, Mandy Elizabeth**
**17 Meadow Rise Iwade**
**Sittingbourne Kent ME9 8SB(GB)**

(74) Representative: **Hunter, Keith Roger Ian et al,**
**4 York Road**
**London SE1 7NA(GB)**

(54) **Benzothiazinone derivatives.**

(57) The invention provides fungicidal compositions containing benzothiazinone derivatives of formula:-

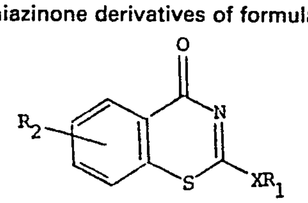

I

wherein X is O, S or NH; $R_2$ represents a hydrogen atom or an alkyl group and $R_1$ represents an optionally substituted alkyl, alkenyl, heterocyclic, or aryl group; certain novel benzothiazinone compounds, a process for the preparation of such compounds and a method of combating plant pathogenic fungi using such compositions or compounds.

EP 0 245 902 A2

Croydon Printing Company Ltd

T 515 FF

BENZOTHIAZINONE DERIVATIVES

This invention relates to fungicidal compositions containing benzothiazinone derivatives, certain novel benzothiazinone compounds, a process for the preparation of such compounds and a method of combating plant pathogenic fungi using such compositions or compounds.

UK Patent Specification No. 1,065,920 describes 2-substituted benzoxazinones of the formula:-

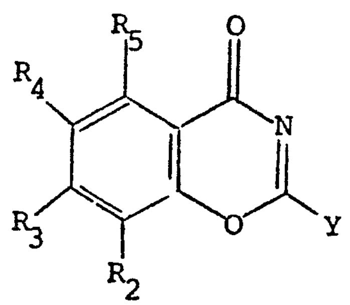

wherein each of $R_2$-$R_5$ denotes, inter alia, hydrogen, halogen, alkyl; and Y represents $OR_6$, $SR_8$ or $NR_7R_8$, $R_6$-$R_8$ being various hydrocarbon groupings, and discloses the activity of such compounds against certain fungi. The derivatives of this structure in which $R_2$, $R_3$, $R_4$ and $R_5$ all represent hydrogen atoms, Y represents a phenoxy group, a naphthyloxy group or a group $-OCH_2CCl_3$ and the heterocyclic oxygen atom is replaced by a sulphur atom are described in German Auslegeschrift 1302657 but there is no disclosure in that document of any fungicidal activity in the compounds concerned.

It has now been discovered that useful fungicidal activity is present in certain 2-substituted benzothiazinones, some of which are novel. According to the present invention there is therefore provided a fungicidal composition which comprises a carrier and, as active ingredient, a 2-substituted benzothiazinone of the general formula I:

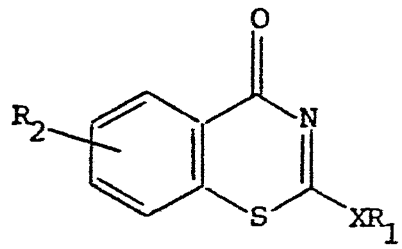

I

wherein X represents an oxygen or sulphur atom or the group NH, $R_2$ represents a hydrogen atom or an alkyl group, and $R_1$ represents an optionally substituted alkyl, alkenyl, heterocyclic or aryl, preferably phenyl, group.

When any of the foregoing substituents are designated as being optionally substituted, the substituent groups which are optionally present may be any one or more of those customarily employed in the development of pesticidal compounds, and/or the modification of such compounds to influence their structure/activity, persistence, penetration or other property. Specific examples of such substituents include halogen, especially fluorine, chlorine or bromine, atoms, and nitro, cyano, alkyl, haloalkyl, especially trifluoromethyl, alkoxy, alkylthio, alkanoyl, alkylsulphinyl, alkylsulphonyl and alkoxycarbonylalkoxy groups and phenoxy groups optionally substituted by one or more halogen atoms or haloalkyl groups. When any of the foregoing substituents represents or contains an alkyl or alkenyl substituent group, this may be linear or branched and may contain up to 12, preferably up to 6, and especially up to 4, carbon atoms, suitable examples being methyl, ethyl, propyl, butyl and propenyl. When they represent an aryl group this is preferably a phenyl group and, when they

represent a heterocyclic group, this is preferably a pyridyl group.

Preferably X represents an oxygen or sulphur atom; $R_2$ represents a hydrogen atom or a methyl group; and $R_1$ represents an alkyl group of 1 to 4 carbon atoms, an allyl group, a pyridyl group, or a phenyl group optionally substituted by 1 or 2 substituents selected from fluorine and chlorine atoms, nitro and cyano groups, methyl, propyl, trifluoromethyl, methoxy, methylthio, acetyl, methylsulphinyl, methylsulphonyl, methoxycarbonylethoxy and chloro-trifluoromethyl-phenoxy groups.

Many of the compounds of formula I are novel, and the invention therefore also extends to these novel compounds <u>per se</u>. The novel compounds are the benzothiazinone compounds of formula I in which the substituents are as defined above, with the proviso that, when $R_1$ is a phenyl, naphthyl or 2,2,2-trichloroethyl group, and $R_2$ is hydrogen, then X is sulphur or NH.

The invention also provides a process for the preparation of compounds of the general formula I defined above, which comprises reacting a reagent of formula II

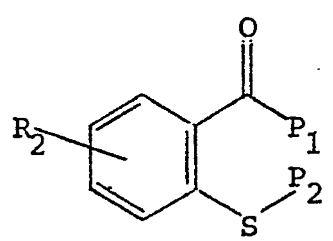

II

with a compound of the general formula III

$R_1X$-Q                III

wherein X, $R_1$ and $R_2$ are as defined above and, $P_1$ represents a group $OR_3$ in which $R_3$ represents a hydrogen atom or an alkyl group, $P_2$ represents a hydrogen atom and Q represents a cyano group; or $P_1$ and $P_2$ together represent a group -N=C-
Hal

in which Hal represents a halogen atom, and Q represents a hydrogen atom. The reaction is conveniently carried out in an inert organic solvent, such as ether or dichloromethane at a temperature from room temperature to reflux temperature. When $P_1$ and $P_2$ together represent a group -N=C-
|
Hal

and Q represents a hydrogen atom, it is preferred that the reaction be carried out in the presence of a base, suitably an organic base such as a trialkylamine, triethylamine being most preferred.

The starting compound of formula II in which $P_1$ and $P_2$ together represent a group -N=C-
|
Hal

may conveniently be prepared from anthranilic acid by reaction with acid/nitrite, followed by thiocyanate to form 2-thiocyanato-benzoic acid, which is then reacted with phosphorus pentachloride, suitably in an organic solvent such as an ether.

The starting compound of formula III in which Q represents a cyano group may be conveniently prepared by reacting a compound of formula $R_1XOH$ in which $R_1$ and X are as defined above with cyanogen bromide, suitably in an organic solvent such as acetone and, preferably, in the presence of an organic base such as triethylamine.

The invention includes also a method for making a fungicidal composition as defined above which comprises bringing a compound of formula I into association with at least one carrier.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be

used. Preferably compositions according to the invention contain 0.5 to 95% by weight of active ingredient.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic

amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active ingredient. Emulsifiable concentrates usually

contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

Of particular interest in enhancing the duration of the protectant activity of the compounds of this invention is the use of a carrier which will provide a slow release of the fungicidal compounds into the environment of the plant which is to be protected. Such slow-release formulations could, for example, be inserted in the soil adjacent to the roots of a vine plant, or could include an adhesive component enabling them to be applied directly to the stem of a vine plant.

The invention still further provides the use as a fungicide of a benzothiazinone of the general formula I as defined above, and a method for combating fungus at a locus, which comprises treating the locus, which may for example be plants subject to

or subjected to fungal attack, seeds of such plants or the medium in which such plants are growing or are to be grown, with such a derivative.

The present invention is of wide applicability in the protection of crop plants against fungal attack. Typical crops which may be protected include vines, grain crops such as wheat and barley, rice, beans and apples. The duration of protection is normally dependent on the individual compound selected, and also a variety of external factors, such as climate, whose impact is normally mitigated by the use of a suitable formulation.

The invention is illustrated in the following Examples.

Example 1

A) Preparation of 2-thiocyanato-benzoic acid

A solution of sodium nitrite (16.5g, 0.2mol) in water (20ml) was added dropwise to a stirred suspension of anthranilic acid (30g 0.2ml) in 1:1 concentrated HCl: water (90ml). The mixture was cooled in an ice bath. The resulting brown solution was added dropwise to a stirred mixture of potassium thiocyanate (63g, 0.65mol) and copper (I) thiocyanate (21.3g, 0.18mol) in water (50ml) at 70°C. The brown mixture was cooled and filtered, then the residue was extracted with acetone. The acetone was removed in vacuo to leave the desired product as a brown/yellow solid, m pt 152-153°C.

B) Preparation of 2-chloro-1,3-benzothiazin-4-one

2-thiocyanato-benzoic acid, prepared as in A (33.6g, 0.19ml), was stirred with phosphorus pentachloride (40g, 0.192ml) in di-n-butyl ether (200ml) under nitrogen at 70°C for six hours. The mixture was cooled and the brown solid was filtered and washed with ether, to yield the desired product, m pt. 112°C.

C) Preparation of 2-(4-chlorophenoxy)-1,3-benzothiazin-4-one

A solution was prepared by adding triethylamine (2.8ml, 0.02mol) to 4-chlorophenol (2.56g, 0.20mol) in dichloro -methane, then this solution was added dropwise to a stirred

suspension of 2-chloro-1,3-benzothiazin-4-one (4g, 0.20mol) in dichloromethane. The resulting mixture was stirred for 18 hours, then water was added. The organic layer was dried over magnesium sulphate, then concentrated using a rotary evaporator. The resulting brown solid was purified by flash chromatography using 3% methanol in dichloromethane, to yield the desired final product m.pt. 114-115°C.

| Analysis | Calc | C 58.0; | H 2.8; | N 4.8% |
|---|---|---|---|---|
| | Found | C 58.0; | H 2.9; | N 5.1% |

Example 2

A) Preparation of 2-methyl-4-chlorophenyl cyanate

4-chloro-2-methyl phenol (10g, 0.07 mol) and cyanogen bromide (7.4g, 0.07 mol) were stirred in acetone at 0°C and triethylamine (9.7 ml, 0.07 mol) was added dropwise. The mixture was stirred at 0°C for a further 2 hours, then filtered. The filtrate was concentrated using a rotary evaporator to leave the desired product as a fawn solid. This was used without further purification.

The melting point of the product, after recrystallisation from ethyl acetate/hexane, was found to be 60°C.

| Analysis | Calc. | C 57.3; | H 3.6; | N 8.4% |
|---|---|---|---|---|
| | Found | C 57.6; | H 3.3; | N 7.6% |

B) Preparation of 2-(4-chloro-2-methyl)phenoxybenzothiazinone

2-methyl-4-chlorophenyl cyanate (1.8g, 0.01 mol) and ethyl 2-mercaptobenzoate (2g, 001 mol) were refluxed in ether (30ml) for 72 hours. The desired product was obtained by filtration as a white solid, m.pt. 144°C (recrystallised from ethanol)

| Analysis | Calc. | C 59.3; | H 3.3; | N 4.6% |
|---|---|---|---|---|
| | Found | C 59.8; | H 3.2; | N 4.4% |

Example 3 - 43

Following procedures similar to those described in Examples 1 and 2 above, substituting where necessary the chlorophenol of Example 1C or the 4-chloro-2-methylphenol of Example 2A with the appropriate thiophenol or aniline, further 2-substituted

benzothiazinones were prepared, whose physical characteristics and analyses are given in Table I below. In this table, the compounds are identified by reference to the substituents in the following formula:-

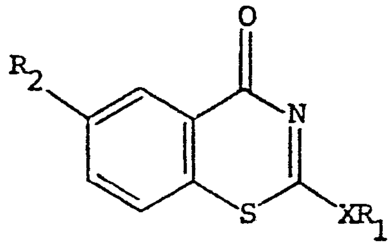

I

Table I

| Example No. | X | $R_2$ | $R_1$ | M.Pt.°C | Analysis C Calc. | C Found | H Calc. | H Found | N Calc. | N Found |
|---|---|---|---|---|---|---|---|---|---|---|
| 3 | O | H | Phenyl | 111-112 | 65.8 | 65.5 | 3.5 | 3.5 | 5.5 | 5.6 |
| 4 | O | H | 4-F Phenyl | 144-145 | 61.5 | 61.1 | 2.9 | 2.6 | 5.1 | 5.1 |
| 5 | O | H | 3-F Phenyl | 134-135 | 61.5 | 60.6 | 2.9 | 3.0 | 5.1 | 5.2 |
| 6 | O | H | 2-F Phenyl | 150-151 | 61.5 | 60.3 | 2.9 | 3.0 | 5.1 | 5.3 |
| 7 | O | H | 4-$NO_2$ Phenyl | 198-200 | 56.0 | 55.7 | 2.7 | 2.9 | 9.3 | 9.3 |
| 8 | O | H | 3-$NO_2$ Phenyl | 195 | 56.0 | 56.4 | 2.7 | 2.8 | 9.3 | 9.5 |
| 9 | O | H | 2-$NO_2$ Phenyl | 191-192 | 56.0 | 56.4 | 2.7 | 2.7 | 9.3 | 9.8 |
| 10 | O | H | 4-$CH_3S$ Phenyl | 155 | 59.8 | 59.4 | 3.6 | 3.7 | 4.6 | 4.8 |
| 11 | O | H | 4-CN Phenyl | 230 | 64.3 | 64.3 | 2.9 | 2.8 | 10.0 | 10.0 |
| 12 | O | H | 4-$COCH_3$ Phenyl | 199 | 64.6 | 64.1 | 3.7 | 3.5 | 4.7 | 4.8 |
| 13 | O | H | 4-$CH_3SO$ Phenyl | 165 | 56.8 | 56.1 | 3.5 | 3.6 | 4.4 | 5.0 |
| 14 | O | H | 4-$CH_3SO_2$ Phenyl | 244-245 | 54.0 | 53.5 | 3.3 | 3.3 | 4.2 | 4.6 |
| 15 | O | H | 4-$OCH(CH_3)COOCH_3$ Phenyl | 134-135 | 60.5 | 60.4 | 4.2 | 4.3 | 3.9 | 4.2 |
| 16 | O | $CH_3$ | 2-F Phenyl | 164-165 | 62.7 | 62.5 | 3.5 | 3.7 | 4.9 | 5.1 |
| 17 | O | $CH_3$ | 4-Cl Phenyl | 164 | 59.3 | 59.1 | 3.3 | 3.6 | 4.6 | 5.0 |

Table I (Con't)

| Example No. | X | $R_2$ | $R_1$ | M.Pt.°C | Analysis C Calc. | C Found | H Calc. | H Found | N Calc. | N Found |
|---|---|---|---|---|---|---|---|---|---|---|
| 18 | O | $CH_3$ | 4-$COCH_3$ Phenyl | 187-189 | 65.6 | 65.1 | 4.2 | 4.2 | 4.5 | 4.9 |
| 19 | O | $CH_3$ | 4-$NO_2$ Phenyl | 261-263(dec) | 57.3 | 57.4 | 3.2 | 3.4 | 8.9 | 9.1 |
| 20 | S | H | 4-Cl Phenyl | 192 | 55.0 | 54.8 | 2.6 | 2.6 | 4.6 | 4.8 |
| 21 | S | $CH_3$ | 4-Cl Phenyl | 214 | 56.3 | 56.4 | 3.1 | 3.6 | 6.4 | 5.7 |
| 22 | S | $CH_3$ | 4-$CH_3$ Phenyl | 187 | 64.2 | 63.2 | 4.4 | 4.5 | 4.7 | 5.0 |
| 23 | NH | H | $C_3H_7$(iso) | 152-153 | 60.0 | 59.9 | 5.4 | 5.5 | 12.7 | 12.8 |
| 24 | NH | $CH_3$ | $CH_3$ | 228-230 | 58.3 | 58.9 | 4.9 | 4.8 | 13.6 | 13.8 |
| 25 | NH | $CH_3$ | $C_3H_7$(iso) | 156-158 | 61.5 | 61.0 | 6.0 | 6.0 | 11.9 | 12.0 |
| 26 | NH | $CH_3$ | $C_4H_9$(iso) | 162-164 | 62.9 | 62.3 | 6.5 | 6.5 | 11.3 | 11.9 |
| 27 | NH | $CH_3$ | Allyl | 184-186 | 62.1 | 61.2 | 5.2 | 5.1 | 12.1 | 12.1 |
| 28 | NH | H | 2-F Phenyl | 178-179 | 61.7 | 61.7 | 3.3 | 3.4 | 10.3 | 10.3 |
| 29 | NH | H | 3-Cl-Phenyl | 185-186 | 58.2 | 57.5 | 3.1 | 3.1 | 9.7 | 9.7 |
| 30 | NH | H | 4-Cl Phenyl | 215-216 | 58.2 | 58.2 | 3.1 | 3.0 | 9.7 | 9.7 |
| 31 | NH | H | 4-$CH_3$ Phenyl | 194-195 | 67.1 | 67.1 | 4.5 | 4.6 | 10.6 | 10.9 |
| 32 | NH | H | 4-$C_3H_7$ Phenyl | 171-179 | 68.9 | 68.6 | 5.4 | 5.5 | 9.4 | 9.0 |

Table I (Con't)

| Example No. | X | $R_2$ | $R_1$ | M.Pt. °C | Analysis C Calc. | C Found | H Calc. | H Found | N Calc. | N Found |
|---|---|---|---|---|---|---|---|---|---|---|
| 33 | NH | H | 3-$CF_3$ Phenyl | 171-172 | 55.9 | 55.7 | 2.8 | 2.7 | 8.7 | 8.5 |
| 34 | NH | H | 4-(2-Cl,4-$CF_3$ Phenoxy) Phenyl | 222-224 | 56.2 | 56.1 | 2.6 | 2.5 | 6.2 | 6.0 |
| 35 | O | H | 2-Cl Phenyl | 152-153 | 58.0 | 58.1 | 2.8 | 3.0 | 4.8 | 5.1 |
| 36 | O | H | 2,4-$(NO_2)_2$ Phenyl | 220 | 48.7 | 48.5 | 2.0 | 2.1 | 12.2 | 12.1 |
| 37 | O | H | 2-$CF_3$ Phenyl | 144 | 55.7 | 55.6 | 2.5 | 2.1 | 4.3 | 3.7 |
| 38 | O | H | 4-$CF_3$ Phenyl | 148-149 | 55.7 | 55.8 | 2.5 | 2.7 | 4.3 | 4.6 |
| 39 | O | H | 2-$CH_3O$ Phenyl | 185-186 | 63.2 | 62.9 | 3.9 | 3.9 | 4.9 | 5.1 |
| 40 | O | H | 4-$CH_3O$ Phenyl | 143 | 63.2 | 62.8 | 3.9 | 3.6 | 4.9 | 4.6 |
| 41 | O | H | 2-Pyridyl | 220 | 60.9 | 60.9 | 3.1 | 3.0 | 10.9 | 10.7 |
| 42 | S | H | 4-F Phenyl | 189-190 | 58.1 | 57.7 | 2.8 | 2.8 | 4.8 | 5.0 |
| 43 | S | H | 4-$NO_2$ Phenyl | 198-199 | 53.2 | 53.1 | 2.5 | 2.7 | 8.9 | 9.3 |

Example 44

The fungicidal activity of representative compounds of the invention was evaluated by means of the following tests:-

a) Direct protectant activity against vine downy mildew (Plasmopara viticola; P.v.p)

The test is a direct protectant one using a foliar spray. The lower surfaces of leaves of whole vine plants (cv Cabernet Sauvignon) are sprayed with a solution of the test compound in 1:1 water/acetone containing 0.04% "TWEEN 20" (Trade Mark; a polyoxyethylene sorbitan ester surfactant). The spraying is carried out using a moving track sprayer giving an application rate of 1kg/ha, and after a subsequent 24 hours under normal glasshouse conditions the lower surfaces of the leaves are inoculated by spraying with an aqueous solution containing $10^4$ zoosporangia/ml. The inoculated plants are kept for 24 hours in a high humidity compartment, 5 days under normal glasshouse conditions and then returned for a further 24 hours to high humidity. Assessment is based on the percentage of leaf area covered by sporulation compared with that on control leaves.

b) Direct protectant activity against vine grey mould (Botrytis cinerea; Bcp)

The test is a direct protectant one using a foliar spray. The lower surfaces of detached vine leaves (cv Cabernet Sauvignon) are sprayed with the test compound at a dosage of 1kg/ha using a track sprayer as in (a). 24 Hours after spraying the leaves are inoculated with droplets of aqueous suspension containing $10^5$ conidia/ml. After a further 5 days in high humidity the percentage of leaf area covered by disease is assessed.

c) Activity against barley powdery mildew (Erysiphe graminis f.sp. hordei; Eg)

The test is a direct therapeutic one, using a foliar spray. Leaves of barley seedlings, (cv. Golden Promise)

are inoculated by dusting with mildew conidia one day prior to treatment with the test compound. The inoculated plants are kept overnight at glasshouse ambient temperature and humidity prior to treatment. The plants are sprayed with the test compound at a dosage of 1 kilogram of active material per hectare using a track sprayer as described under (a). After drying, plants are returned to a compartment at 20-25°C and moderate humidity for up to 7 days, followed by assessment. Assessment is based on the percentage of leaf area covered by sporulation compared with that on leaves of control plants.

d) Activity against tomato early blight (Alternaria solani; As)

This test measures the contact prophylactic activity of test compounds applied as a foliar spray.

Tomato seedlings (cv Outdoor Girl) are grown to the stage at which the second true leaf is expanded. The plants are treated using a track sprayer as described under (a). Test compounds are applied as solutions or suspensions in a mixture of acetone and water (50:50v/v) containing 0.04% surfactant ("TWEEN 20" - Trademark).

One day after treatment the seedlings are inoculated by spraying the leaf upper surfaces with a suspension of A. solani conidia containing $10^4$ spores/ml. For 3 days after inoculation plants are kept moist in a glasshouse compartment at or near 100% RH and 21°C. Thereafter plants are kept under humid, but not saturated, conditions.

Disease is assessed 7 days after inoculation, based on the density and spread of lesions.

e) Activity against wheat eyespot in-vitro (Pseudocercos porella herpotrichoides; PhI)

This test measures the in vitro activity of compounds against the fungus causing wheat eyespot.

The Test Compound is dissolved or suspended in acetone and is added to molten half strength Potato Dextrose Agar

to give a final concentration of 100ppm compound and 3.5% acetone. After agar has set, plates are inoculated with 6mm diameter plugs of agar/mycelium taken from a 14 day old culture of P. herpotrichoides.

Plates are incubated at 20°C for 12 days and radial growth from the inoculation plug is measured.

f) Activity against Fusarium in-vitro (Fusarium species; FsI)

This test measures the in vitro activity of compounds against a species of Fusarium that causes stem and root rots.

Compound is dissolved or suspended in acetone and added to molten half strength Potato Dextrose Agar to give a final concentration of 100ppm compound and 3.5% acetone. After agar has set, plates are inoculated with 6mm diameter plugs of agar and mycelium taken from a 7 day old culture of Fusarium sp..

Plates are incubated at 20°C for 5 days and radial growth from the plug is measured.

g) Activity against rice leaf blast (Pyricularia oryzae Po)

The test is a direct therapeutic one using a foliar spray. The leaves of rice seedlings (about 30 seedlings per pot) are sprayed with an aqueous suspension containing $10^5$ spores/ml 20-24 hours prior to treament with the test compound. The inoculated plants are kept overnight in high humidity and then allowed to dry before spraying with the test compound at a dosage of 1 kilogram of active material per hectare using a track sprayer as described under (a). After treatment the plants are kept in a rice compartment at 25-30°C and high humidity. Assessments are made 4-5 days after treatment and are based on the density of necrotic lesions per leaf when compared with control plants.

h) Activity against wheat brown rust (Puccinia recondita; Pr)

The test is a direct protectant one using a foliar spray. Wheat seedlings (cv Brigand) are grown to the 1-1½

leaf stage. The plants are then sprayed with the test compound at a dosage of 1 kg/ha using a track sprayer as described under (a). Test compounds are applied as solutions or suspensions in a mixture of acetone and water (50:50 v/v) containing 0.04% surfactant ("TWEEN 20" - Trade Mark).

18-24 hours after treatment, the seedlings are inoculated by spraying the plants from all sides with an aqueous spore suspension containing about $10^5$ spores/ml. For 18 hours after inoculation, the plants are kept in high humidity conditions at a temperature of 20-22°C. Thereafter, the plants are kept in ambient glasshouse conditions, that is, in moderate relative humidity and at a temperature of 20°C.

The disease is assessed 10 days after inoculation on the basis of the percentage of the plant covered by sporulating pustules compared with that on the control plants.

The extent of disease control in all the above tests is expressed as a rating compared with either an untreated control or a diluent-sprayed- control, according to the criteria:-

0 = less than 50% disease control

1 = about 50-80% disease control

2 = greater than 80% disease control

The results of these tests are set out in Table II below:

Table II

| Ex No | Fungicidal Activity |
|---|---|
| 1 | As 2; Pvp 1 |
| 2 | Pr 1 |
| 3 | Pvp 1 |
| 4 | Pvp 1 |
| 5 | As 2; Pvp 1 |
| 6 | Pvp 2; Bcp 1; As 1 |
| 7 | Pvp 2 |
| 8 | Pvp 1; As 2; Eg 1 |
| 9 | Pvp 2; As 2 |
| 10 | Pvp 2; As 1 |
| 11 | Pvp 1; As 2 |
| 12 | Pvp 2 |
| 13 | Bcp 1; Ph 1 |
| 16 | Pvp 1; Fs 1 |
| 18 | Pvp 2; Fs 1 |
| 19 | Po 2 |
| 20 | Pvp 1; As 2 |
| 22 | Ph 1; Fs 1 |
| 23 | As 2 |
| 25 | Pvp 2; Eg 1; Ph 2 |
| 26 | Pvp 1 |
| 30 | As 2 |
| 32 | As 1 |
| 35 | Pvp 1; As 1; Fs 1 |
| 36 | Eg 1 |
| 37 | Pvp 2; Pr 1; Ph 1; Fs 1 |
| 38 | Pvp 1; As 2; Ph 1; Fs 1 |
| 39 | Pvp 1; Fs 1 |
| 40 | Pvp 2; Ph 1; Fs 1 |
| 41 | Bcp 1 |
| 42 | Pvp 2; As 2; Fs 1 |
| 43 | Pvp 2; As 2; Po 1 |

T 515 FF

CLAIMS

1. A fungicidal composition which comprises a carrier and, as active ingredient, a benzothiazinone of general formula I:-

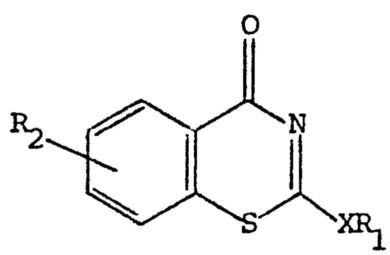

I

wherein X represents an oxygen or sulphur atom or the group NH; $R_2$ represents a hydrogen atom or an alkyl group; and $R_1$ represents an optionally substituted alkyl, alkenyl, heterocyclic or aryl group.

2. A composition as defined in claim 1 wherein the active ingredient is a compound of formula I wherein $R_2$ represents a hydrogen atom or an alkyl group of 1 to 6 carbon atoms; and $R_1$ represents an alkyl or alkenyl group of up to 6 carbon atoms, a pyridyl group, or a phenyl group optionally substituted by one or more substituents selected from halogen atoms, nitro and cyano groups, alkyl, haloalkyl, alkoxy, alkythio and alkanoyl groups of up to 6 carbon atoms, alkylsulphinyl and alkylsulphonyl groups of up to 6 carbon atoms, alkoxycarbonylalkoxy groups of up to 8 carbon

atoms, and phenoxy groups optionally substituted by one or more halogen atoms or haloalkyl groups.

3. A composition as claimed in claim 1 or claim 2 wherein X is an oxygen or sulphur atom.

4. A composition as claimed in any one of claims 1, 2 and 3 wherein $R_2$ represents a hydrogen atom or a methyl group; and $R_1$ represents an alkyl group of 1 to 4 carbon atoms, an allyl group, a pyridyl group, or a phenyl group optionally substituted by 1 or 2 substituents selected from fluorine and chlorine atoms, nitro and cyano groups, methyl, propyl, trifluoromethyl, methoxy, methylthio, acetyl, methylsulphinyl, methylsulphonyl, methoxycarbonylethoxy and chloro-trifluoromethyl-phenoxy groups.

5. A composition as claimed in any one of claims 1, 2, 3, and 4 which comprises at least two carriers, at least one of which is a surface-active agent.

6. A method for making a fungicidal composition as defined in claim 1 which comprises bringing a benzothiazinone compound as defined in claim 1 into association with at least one carrier.

7. A benzothiazinone compound of the general formula I as shown in claim 1, wherein X, $R_1$ and $R_2$ are as defined in claim 1, with the the proviso that, when $R_1$ is a phenyl, naphthyl or 2,2,2-trichloroethyl group and $R_2$ is a hydrogen atom, then X is sulphur or NH.

8. A process for the preparation of a benzothiazinone compound of the general formula I as defined in claim 7 which comprises reacting a compound of the general formula II

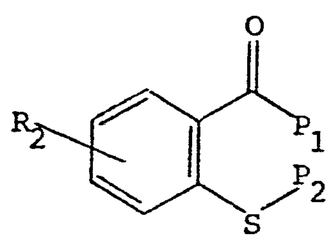

II

0245902

with a compound of the general formula III

$$R_1X\text{-}Q \qquad III$$

wherein X, $R_1$ and $R_2$ are as defined in claim 7 and, $P_1$ represents a group $OR_3$ in which $R_3$ represents a hydrogen atom or an alkyl group, $P_2$ represents a hydrogen atom and Q represents a cyano group; or $P_1$ and $P_2$ together represent a group 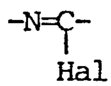

in which Hal represents a halogen atom, and Q represents a hydrogen atom.

9. A process as claimed in claim 8 wherein the reaction is carried out in an inert, organic solvent in the presence of an organic base.

10. A benzothiazinone compound as claimed in claim 7, whenever prepared by a process as claimed in claims 8 or claim 9.

11. A method of combating fungus at a locus, characterised by treating the locus with a fungicidally effective amount of a composition as claimed in any one of claims 1-5 or a compound as claimed in claim 7 or claim 10.

12. A method as claimed in claim 13, wherein the locus comprises plants subject to or subjected to fungal attack, seeds of such plants, or the medium in which the plants are growing or are to be grown.

13. The use as a fungicide of a compound of formula I as defined in any one of claims 1-5, 7 and 10.